# EUROPEAN PATENT APPLICATION

(11) **EP 1 219 232 A1**
(43) Date of publication of application: **03.07.2002**
(21) Application number: 01124387.0
(22) Date of filing: 24.10.2001
(51) Int. Cl.: A61B 5/00

(54) **Spectrophotometric blood glucose determination apparatus and determination method thereof**

(30) Priority: 28.12.2000 JP 2000403518
(71) Applicant: BIOX CORPORATION, Chiyoda-ku, Tokyo (JP)
(72) Inventor: Miki, Keizaburo 548-7, Higashikoiso, Kanagawa (JP); Amano, Toshio c/o Opt. Research Inc., Tokyo (JP); Hoshina, Sadayori, Tokyo (JP)
(74) Representative: Müller-Boré & Partner Patentanwälte

(57) **Abstract**

To provide a portable blood glucose determination apparatus and a determination method for either invasive or non-invasive measurement of glucose concentration in blood by optical observation excelling in measuring accuracy and reproducibility.

A spectrophotometric blood glucose determination apparatus is an infra-red quantitative analysis instrument for measuring concentration of glucose in blood, provided with the following units numbered (1) through (3):
(1) a near-infrared irradiating unit for continuously dividing wavelengths of near-infrared light in a wavelength range of 0.8 to 2.5 µm into fine portions with acousto-optic tunable filter and irradiating the subject of measurement therewith;
(2) a photoelectric conversion unit for receiving and photoelectrically converting lights transmitted by the subject of measurement irradiated therewith by the near-infrared irradiating unit; and
(3) a glucose concentration computing unit for determining the glucose concentration in the blood within the subject of measurement by analyzing an absorbance spectrum obtained on the basis of detection signals resulting from photoelectric conversion by the photoelectric conversion unit,
and a spectrophotometric blood glucose determination method including each step using the above described each unit.

## Description

### FIELD OF THE INVENTION

The present invention relates to a spectrophotometric blood glucose determination apparatus and a spectrophotometric determination method of blood glucose, and more particularly to a near-infrared spectrophotometric blood glucose determination apparatus and a spectrophotometric determination method of blood glucose using the same determination apparatus capable of determining the concentration of glucose in a blood sample, particularly the concentration of glucose in the blood non-invasively from outside the human body without having to draw blood therefrom.

### BACKGROUND OF THE INVENTION

In recent years, in the face of an alarming increase in diabetic patients, there is a keen call for simple, quick and yet accurate blood glucose measuring instruments to obtain blood glucose data required for their treatment. Also, if there are made available blood glucose meters which patients can use safely and easily for themselves, they will make great contributions to the control of blood glucose counts by the patients themselves.

Whereas the blood glucose count can be determined by measuring the concentration of glucose contained in the blood, there have been proposed a number of methods of measurement according to the prior art, including one based on the reducibility of glucose, another using a direct reaction of glucose in an acid condition and still another using an enzymatic reaction of glucose. Methods used in clinical medicine include one by which blood drawn from a finger or a toe or sampled in some other way is reacted with glucose oxidase, the degree of coloring or electric potential is measured by utilizing a reaction dependent on the concentration of glucose in blood, and converting the degree of coloring or electric potential into a blood glucose count.

However, as stated above, every type of blood glucose meters in conventional use require drawing of blood. This means not only that a diabetic patient has to endure the pain of blood extraction, which is required more than 100 times for a patient in a month, but also that considerable time, labor and money have to be spent on sterilization to prevent possible infection at the time of blood extraction.

Furthermore, glucose oxidase used in the aforementioned enzymatic reaction, which is a fundamental technique, is enzymatic protein, which is susceptible to protein denaturation and activity weakening, resulting in a preservation problem that the protein can be maintained for only about six months at most.

Moreover, as this enzymatic reaction system is a method to conjugate the product of the glucose oxidase reaction with a pigment system and measure any change in pigment, the reaction is complex, and many control means are required for setting the conditions of reaction. Accordingly, it needs a costly measuring instrument, and moreover involves the problem of taking as long as 10 to 15 minutes per sample from blood extraction to actual measurement.

In view of this circumstance, there is proposed a method by which the human body is irradiated with a near-infrared light and the blood glucose count is determined according to the intensity of the transmitted light. By any of these methods, however, wavelength resolution is significant, and it is impossible to detect on the spectrum the relative heights of the waveform (peak and trough) which are finely varied by the coupling of glucose and protein, resulting in insufficiency in the accuracy and reproducibility of the blood glucose measurement. None of these methods have become available for practical use.

### SUMMARY OF THE INVENTION

Therefore, with an eye toward obviating the problems of the enzymatic methods in determining the glucose concentration in blood in view of the circumstances noted above, an object of the invention is to provide a spectrophotometric blood glucose determination apparatus, which is a quantitative analysis instrument capable of so-called non-invasive determination, for invasive measurement method of extracted blood samples by optical observation and non-invasive measurement method developed from the invasive, i.e. detection from outside the human body glucose, glycohemoglobin and other contents in the blood and assessing the concentrations of the glucose and others. It is intended to be an apparatus and a method of solving the aforementioned problems, excelling in accuracy and reproducibility.

The present inventors made zealous studies to solve the problems noted above, and took note of the possibility to accomplish the first of these tasks, i.e. making available non-invasive determination, by realizing a quantitative analysis instrument which can irradiate a blood sample or blood in vivo from outside, for example through a finger of a hand, with a light in the near-infrared band and measure the absorbance of wavelengths specific to glucose. The inventors further found that the second of the tasks, i.e. determination with high accuracy and reproducibility, could be accomplished by irradiating the part of a human body to measure with a near-infrared light divided into fine portions, receiving the light transmitted by tissues of the human body including its skin, flesh and capillaries, analyzing the absorbance spectrum by switching the wavelength of the irradiating light, and computing the glucose concentration in the blood. The inventors were able to complete the invention on the basis of these findings.

Thus, the present invention relates to a spectrophotometric blood glucose determination apparatus, which is an infra-red quantitative analysis instrument for measuring the concentration of glucose in blood, provided with the following means numbered (1) through (3):
(1) near-infrared irradiating means for continuously dividing the wavelengths of near-infrared light in the wavelength range of 0.8 to 2.5 µm into fine portions with an acousto-optic tunable filter and irradiating the subject of measurement therewith;
(2) photoelectric conversion means for receiving and photoelectrically converting the lights transmitted by the subject of measurement irradiated therewith by the near-infrared irradiating means; and
(3) glucose concentration computing means for determining the glucose concentration in the blood within the subject of measurement by analyzing the absorbance spectrum obtained on the basis of detection signals resulting from photoelectric conversion by the photoelectric conversion means.

A second aspect of the invention relates to a spectrophotometric blood glucose determination method permitting determination of the concentration of glucose in blood, comprising:
1) a near-infrared irradiating step of continuously dividing wavelengths of near-infrared light in a wavelength range of 0.8 to 2.5 µm into fine portions with an acousto-optic tunable filter and irradiating a subject of measurement therewith;
2) a photoelectric conversion step of receiving and photoelectrically converting the lights transmitted or reflected by the subject of measurement irradiated therewith at the near-infrared irradiating step; and
3) a glucose concentration computing step of assessing the glucose concentration in the blood within the subject of measurement by analyzing and computing an absorbance spectrum obtained on the basis of detection signals resulting from photoelectric conversion at the photoelectric conversion step.

### BRIEF DESCRIPTION OF THE DRAWINGS

FIG. 1 is a block diagram illustrating a configuration of a spectrophotometric blood glucose determination apparatus according to the present invention;
FIG. 2 shows an external perspective view of a portable spectrophotometric blood glucose determination apparatus according to the invention;
FIG. 3 is an expanded illustration of a fixing tool for the subject of measurement of a spectrophotometric blood glucose determination apparatus according to the invention;
FIG. 4 shows a partial section of the subject of measurement fixing tool shown in FIG. 3 as viewed in the directions of the arrows (a) of the X-X' line;
FIG. 5 shows a partial section of the subject of measurement fixing tool shown in FIG. 3 as viewed in the directions of the arrows (b) of the X-X' line;
FIG. 6 is an expanded illustration of another fixing tool for the subject of measurement of a spectrophotometric blood glucose determination apparatus according to the invention;
FIG. 7 shows a partial section of the subject of measurement fixing tool shown in FIG. 6 as viewed in the directions of the arrows (a) of the X-X' line;
FIG. 8 shows a partial section of the subject of measurement fixing tool shown in FIG. 6 as viewed in the directions of the arrows (b) of the X-X' line;
FIG. 9 shows a near-infrared absorbance spectrum obtained by measurement with a first embodiment;
FIG. 10 shows an expanded near-infrared absorbance spectrum of b - a = c with the tool of FIG. 6; and

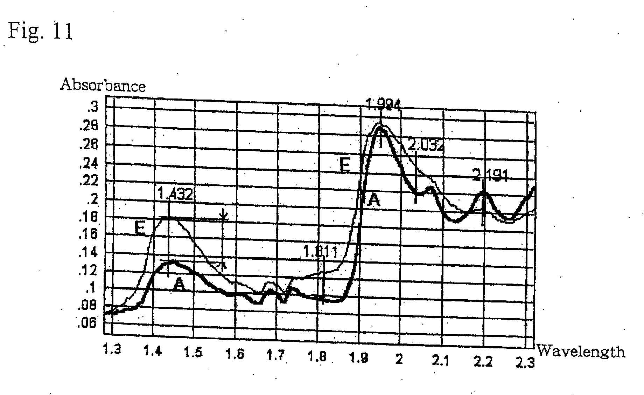

FIG. 11 shows near-infrared absorbance spectra obtained by measurement of A and E with a second embodiment.

### DETAILED DESCRIPTION OF THE PREFERRED EMBODIMENTS

The present invention will be described in detail below.

Near-infrared irradiating means of a spectrophotometric blood glucose determination apparatus according to the invention continuously divides the wavelengths of near-infrared light in the wavelength range of 0.8 to 2.5 µm into fine portions and irradiates the subject of measurement therewith.

The near-infrared light can be obtained by wavelength selection of lights from a prescribed light source, and can be secured with a spectral analyzer provided with a rotational interference filter or the like permitting the setting of operating conditions as desired. Particularly preferable is near-infrared light dividing means having as one of its constituent elements an acousto-optic variable oscillation tunable filter.

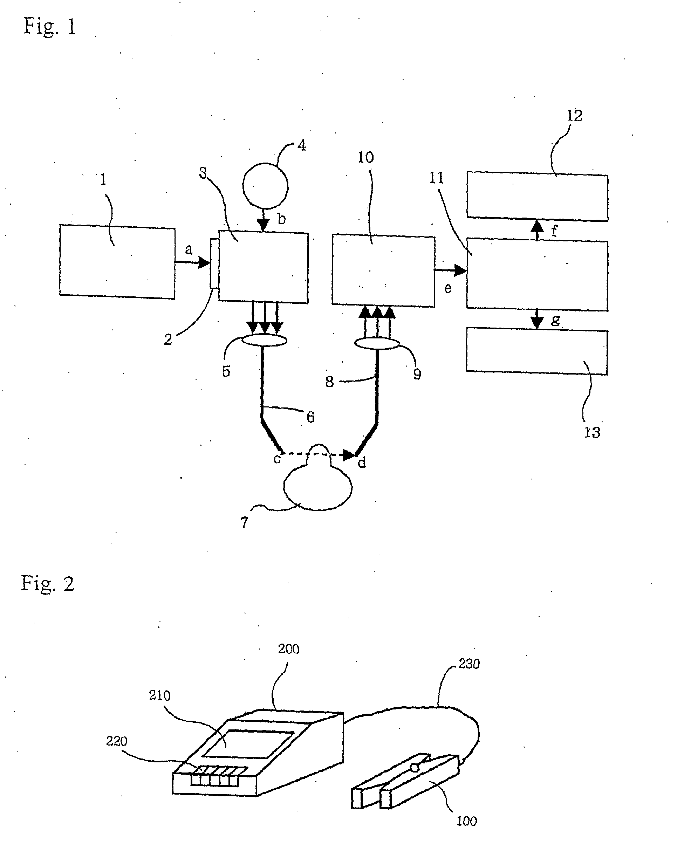

The near-infrared irradiating means provided with the near-infrared light dividing means having as one of its constituent elements the acousto-optic variable oscillation filter is composed of: (i) a light source, (ii) an acousto-optic variable oscillation filter on which light comes incident from the light source, (iii) a high frequency vibrator for applying acoustic vibration to the acousto-optic variable oscillation filter and (iv) a high frequency generating unit for applying a high frequency to the high frequency vibrator. More specifically, as illustrated in FIG. 1, it is composed of a high frequency electric power source 1, a high frequency vibrator 2, an acousto-optic variable oscillation filter 3 and a light source 4.

The high frequency generating unit 1 may be any such unit in ordinary use, not limited to any particular type, and anything capable of generating a high frequency controllable as desired can be used. As the high frequency vibrator 2, which may be anything that can give acoustic vibration to the acousto-optic variable oscillation filter 3, a piezo element is used. The high frequency to be applied to the piezo element may be controlled so as to divide the near-infrared light of 0.8 to 2.5 µm in wavelength range, though partly depending on the type of the medium and the performance of the acousto-optic variable oscillation filter, and the preferable wavelength range is 30 to 100 MHz, particularly 30 to 80 MHz. As the light source 4, a tungsten-halogen lamp or the like is used, but the choice is not limited to them.

The aforementioned near-infrared irradiating means can be operated by a near-infrared irradiating step to be described below. Thus here is provided the spectrophotometric blood glucose determination method, as set forth in Claim 10, wherein the near-infrared irradiating step is a combination of:
(i) a sub-step of applying a high frequency onto a high frequency vibrator;
(ii) a sub-step at which the high frequency vibrator to which the high frequency was applied at the sub-step (i) applies acoustic vibration to an acousto-optic variable oscillation filter; and
(iii) a sub-step of bringing light incident on the acousto-optic variable oscillation filter, to which acoustic vibration was applied at the sub-step (ii), from a light source, and causing near-infrared light in a wavelength range of 0.8 to 2.5 µm to be emitted.

The medium of the acousto-optic variable oscillation filter 3 consists of a birefringent crystal spectral material. In the acousto-optic variable oscillation filter, when acoustic vibration is applied to the birefringent crystal, there arise periodic variations in density, and variations in refractive index due to the variations in density propagate undulatingly in the direction of the acoustic vibration. Therefore, when light comes incident there, some rays are reflected according to the refractive index of each wave face. The filter is designed to give rise to differences in path length among the reflected rays and to emit near-infrared light.

Whatever appropriate birefringent crystal spectral material can be used as desired without limitation to any particular type and, for measuring glucose in blood, a birefringent crystal spectral material capable of emitting finely divided irradiating lights in the wavelength range of 0.8 to 2.5 µm can be selected. For instance, the conceivable choice comprises tellurium dioxide (TeO₂), lithium niobate (LiNbO₃), lithium thallate (LiTaO₃), gallium phosphide (GaP), lead molybdate (PbMoO₄), germanium (Ge), indium posphide (InP), thallium arsenic selenide (Tl₃AsSe₃), silica glass (SiO₂), calcite (CaCO₃) and water(H₂O). It is preferable to choose what is so controlled in the type of material and composition that lights deriving from fine division of near-infrared light wavelengths can be obtained, and tellurium dioxide is particularly preferable.

Irradiation of a human body with near-infrared light obtained by the use of such a birefringent crystal spectral material can form an absorbance spectrum effective for the computation of glucose concentration. Acousto-optic variable wavelength filters suitable for use in a spectrophotometric blood glucose determination apparatus according to the invention include, for instance, acousto-optic tunable filters (AOTF) described in USP 5,120,961 and National Publication of Translation No. 10-512678. Further to avoid temperature drifting of any such acousto-optic variable oscillation filter, the method described in Japanese Patent Laid-Open No. 10-38690 can be used.

For use in irradiation, near-infrared light in the wavelength range of 0.8 to 2.5 µm is suitable, and any shorter wavelength would be unsuitable for analysis because the signal level for measuring glucose absorbance would be too low. On the other hand, any longer wavelength would give rise to a problem of too strong absorbance and a resultant lack of transmissivity.

The lights resulting from the continuous fine division of the near-infrared light in the wavelength range of 0.8 to 2.5 µm for the near-infrared irradiating means with which to irradiate the subject of measurement should preferably be no less fine than 0.001 µm and no rougher than 1 nm in wavelength resolution. The use of so finely divided lights for irradiation makes possible multiple point measurement, which could identify various versions of glucose in blood present as a number of denatured products varying with the form of coupling with protein and absorbing light in different wavelengths. As a result of successful tracking of the phenomenon of absorbance by denatured versions by this multiple point measurement, comprehensive analysis became possible and so did accurate computation of glucose concentrations in blood. At least 200, for instance, or usually several hundreds of measuring points can be selected.

Next will be described the methods of irradiation with near-infrared light by the near-infrared light irradiating means.

Irradiation can be accomplished by one of the following three methods.
(1) First method: The subject of measurement is irradiated with the near-infrared light, and the light transmitted by the subject of measurement is directly focused on a light receiving element.
(2) Second method: The subject of measurement is irradiated with the near-infrared light; the light transmitted by the subject of measurement is reflected by a reflector plate installed on the back side of the subject of measurement; and the light transmitted again by the subject of measurement is focused on a light receiving element.
(3) Third method: The subject of measurement is irradiated with the near-infrared light, and the light transmitted by the subject of measurement is diffusively reflected by a reflector plate installed on the back side of the subject of measurement to be focused on a light receiving element.

While any of these methods can be adopted, the first transmission method is simpler than the others both in hardware configuration and in operation.

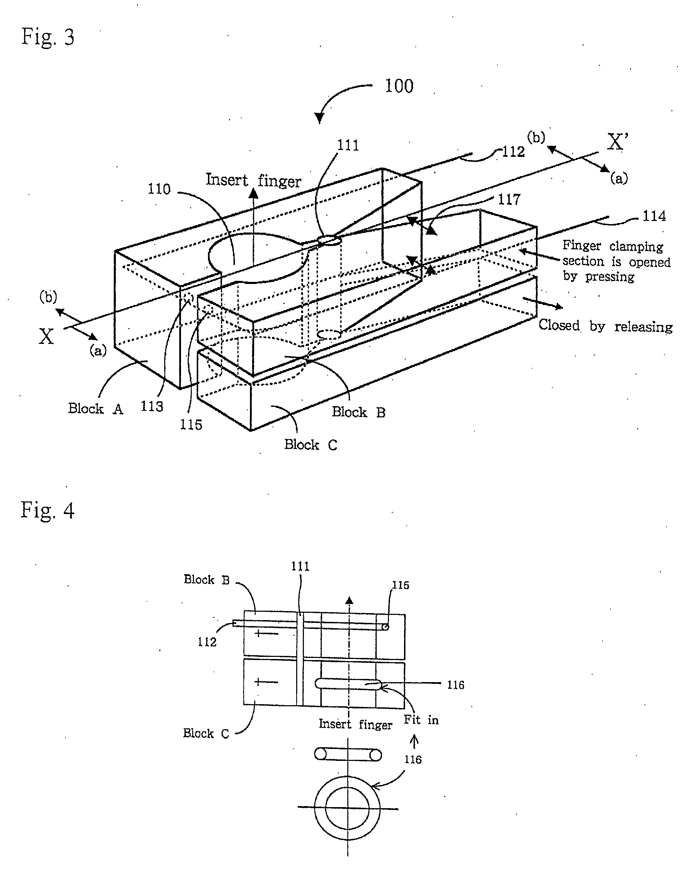

According to the first transmission system, more specifically, in the configuration of a spectrophotometric blood glucose determination apparatus pertaining to the invention, a subject of measurement 7 is irradiated with irradiating light c, and the transmitted light is obtained as received light d as illustrated in FIG. 1. A clipper shown in FIG. 3 is another specific example of the transmission system.

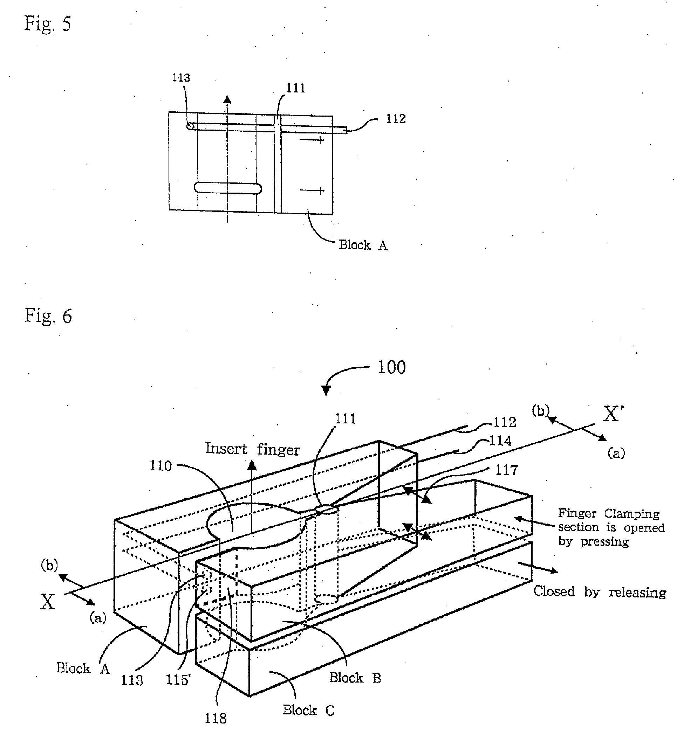

In a specific example of implementing the second transmissive reflection method shown in FIG. 6, the subject of measurement is irradiated from an irradiating spot 113; the light transmitted by the subject of measurement is reflected by a reflector plate installed on the back side of the subject of measurement; and the light transmitted again by the subject of measurement is focused from a light receiving spot 114' on a light receiving element. The material of the reflector plate in the transmissive reflection method may be, though not limited to, ceramic with particular preferability, or else alumina, silica, silicon nitride or the like.

By the third diffusive reflection method, light transmitted by the subject of measurement is diffusively reflected by a reflector plate installed on the back side of the subject of measurement to be focused on a light receiving element.

Incidentally, FIGS. 3 through 8 illustrate tools for non-invasive measurement. For invasive measurement, either a quartz glass cuvette filled with a blood sample drawn from a human body or a reflector plate on which a blood sample is placed can be used. If the extracted blood is either fully or partly dried on the reflector plate, the water content of the blood will evaporate and the absorbance by water molecules in the near-infrared wavelength region will decrease. As a result, the absorbance by glucose in the blood can be determined more clearly and accurately without being disturbed by the absorbance by water.

Next will be described fixing means for the subject of measurement.

What can be used as the subject of measurement include a blood sample drawn from a human body and a portion of a human body, such as a finger, toe, earlobe or any other portion where there are capillaries.

When a human body is to be irradiated with divided near-infrared lights using near-infrared irradiating means according to the invention, it is preferable to use some means for fixing the portion to be measured. Especially in non-invasive measurement from outside the body, it is preferable from the viewpoints of accuracy and reproducibility to pick up two kinds of measured data, one in a normal state of blood circulation and the other in a lightly hemostatic state, and to use the difference between the two sets of data. The fixing means illustrated in FIGS. 3 through 9 would prove useful for determining the difference.

Thus, the difference between the "normal state" and the "hemostatic state" represents the degree of absorbance by blood itself whose quantity in that portion is greater than usual as a result of the congestion of blood. It is cleared of the counts of absorbance by skin and flesh, which would obstruct analysis of the glucose concentration in blood, and useful for enhancing the accuracy of blood measurement.

Each of these means for fixing the subject of measurement is provided with a clamping device capable of creating a state of blood congestion in the portion of the human body to be measured.

As specific examples of fixing means, clippers illustrated in FIGS. 3 through 8 can be cited. The clippers can be used in a state of being. connected to a blood glucose determination apparatus itself by an optical fiber as shown in FIG. 2. The clippers shown in FIGS. 3 through 5 are embodiments of the transmission method, and those in FIGS. 6 through 8 are embodiments of the second transmissive reflection method, both for application to a finger as the subject of measurement. The clippers shown in FIG. 3 are shaped like a pinch in the plan, and consist of blocks A, B and C and finger clamping sections provided on the blocks. The blocks are linked to one another to enable the finger clamping sections to open and close pivoting on a hinge 111, so that the finger clamping sections can be opened by pressing the tips of blocks B and C from outside at the same time. An optical fiber 112 is embedded in block A for use in irradiation, and the block is provided with an irradiating spot 113. A light receiving spot 114 for receiving transmitted light and an optical fiber 115 for delivering light to a light receiving element are embedded in block B. A hemostatic ring 116 is fitted to the finger clamping section of block C. A preferable material for the hemostatic ring is, for instance, sponge.

FIGS. 6 through 8 illustrate a pair of clippers for use in the transmissive reflection method. This pair of clippers differs from the clippers shown in FIGS. 3 through 5 in that an irradiating optical fiber 112 and a light receiving optical fiber 115 embedded in block A and block B are provided with a reflector plate 118 opposite an irradiating spot 113 and a light receiving spot 114' of block A.

In a measuring procedure using either of the fixing means, first the finger clamping sections of blocks B and C are opened, a finger is inserted between them, block B is closed to clamp part of the epidermis and the flesh of the finger in a thickness of 4 to 6 mm in a normal state of blood circulation, and near-infrared light is passed through the finger to carry out the first stage of measurement. Then, block C is closed, a joint closer to the heart than the clamped part is choked with a belt-shaped or annular hemostatic ring 116 and, after waiting a few seconds till the measured portion is congested with blood, the second stage of measurement is carried out. The measured data obtained in this manner are automatically read into an arithmetic circuit.

The photoelectric conversion means consists of a device to receive the light emitted by the near-infrared irradiating means and transmitted by the subject of measurement and to photoelectrically convert it into detection signals that provide an absorbance spectrum. FIG. 1 illustrates a configuration comprising the subject of measurement 7, an optical fiber 8 for guiding a light d transmitted by the subject of measurement 7, a lens 9 and a light receiving element 10 for performing photoelectric conversion into detection signals.

The photoelectric conversion means can be operated by the following photoelectric conversion step. That is, the photoelectric conversion step is a combination of (i) a sub-step of supplying light transmitted or reflected by the subject of measurement to a light receiving element and (ii) a sub-step at which the light receiving element supplies detection signals giving an absorbance spectrum.

The light receiving element 10 may have, though it is not limited to, a configuration in which a polycrystalline film is formed over, for instance, a ceramic substrate. It should preferably be made of a light receiving material capable of efficiently focusing the transmitted light, such as Pbs or In-Ga-As.

The glucose concentration computing means, intended to analytically compute the absorbance spectrum and convert it into glucose concentrations by the molecular extinction coefficient for glucose, is configured of a computing electronic circuit and an analyzing electronic circuit. As shown in FIG. 1, a detection signal e resulting from photoelectric conversion by the light receiving element 10 is entered into spectral waveform analyzing-computing means 11, and the computing electronic circuit and the analyzing electronic circuit carry out the analyses and computations to be described below.

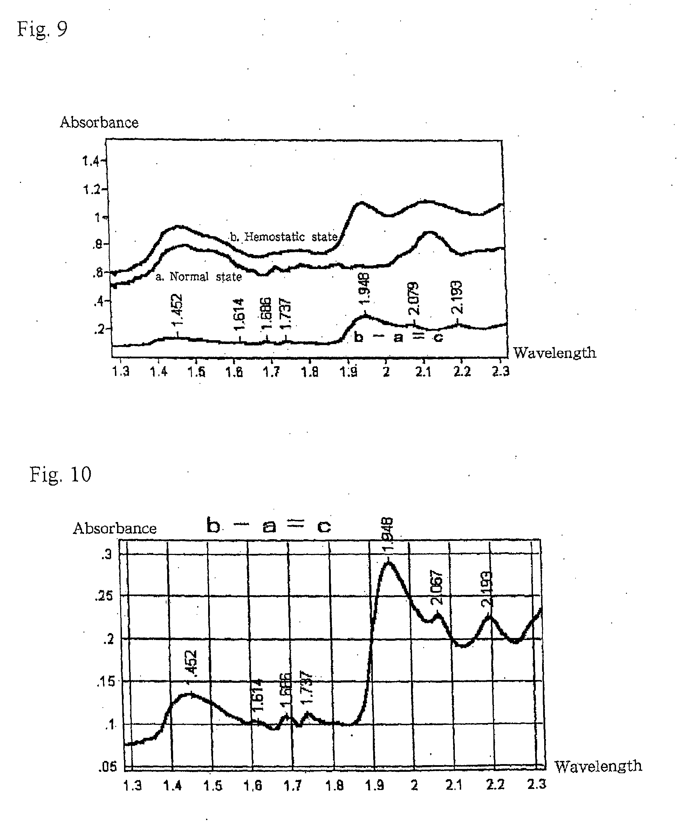

The computing electronic circuit clamps part of the epidermis and the flesh of a human body in a normal state of blood circulation as shown in FIG. 9, carries out the first stage of measurement by passing near-infrared light, chokes the portion and, after waiting till the measured portion is congested with blood, performs the second stage of measurement. The "normal state spectrum" and "hemostatic state spectrum" that have been picked up are subjected to "subtraction" in an image processing manner, i.e. "b. hemostatic state spectrum" - "a. normal state spectrum", to obtain a "blood spectrum" of b - a = c.

In the analyzing electronic circuit, a "blood spectrum database" configured as medical clinical data is stored. As the "blood spectrum database" contains many sets of spectral data including those differing in glucose concentration in blood and those differing in the state of protein-glucose coupling, measurements of glucose concentrations in blood can be obtained by collating and comparing blood spectra obtained by measurement with these data.

The analyzing electronic circuit can also obtain the locations of variants resulting from protein-glucose coupling and identified levels among other items of information in addition to the results of quantitative analysis.

These results of measurement and identification are transferred as signals f and g to numerical display means 12 and numerical telegraphic means 13. Accordingly, it is thereby made possible to display, for instance, numerical values and related information, together with spectra, on the monitor of the blood glucose determination apparatus, or transfer them to prescribed electronic files as required and manage them therein.

In the above-described spectrophotometric blood glucose determination apparatus and the determination method according to the invention, the incorporation of the acousto-optic variable oscillation filter is one of its unique structural features; the filter makes possible rapid measurement, more specifically a measuring speed of a few thousand points per second. Measurement is repeated a few times to a few tens of times at each wavelength and the results are averaged. Also, the set range of wavelengths is scanned a few times to a few tens of times and the results are averaged. This further adds to the reliability of the measured data.

The present invention, which relates to the spectrophotometric blood glucose determination apparatus and the determination method described above, includes as its preferred embodiments the following three, from (1) through (3).
(1) A non-invasive spectrophotometric blood glucose determination apparatus comprising:
   1) near-infrared light dividing means for dividing near-infrared light of 0.8 to 2.5 µm in wavelength, the means consisting of a high frequency generating unit, a piezo element to which a high frequency generated by the high frequency generating unit is applied, an acousto-optic variable oscillation filter to which acoustic vibration is applied from the piezo element, and a light source for supplying light to be brought to incidence on the acousto-optic variable oscillation filter;
   2) near-infrared irradiating means for irradiating a human body with the near-infrared light;
   3) photoelectric conversion means for receiving with a light receiving element and photoelectrically converting the light transmitted by the human body irradiated with the near-infrared light; and
   4) glucose concentration computing means for assessing a glucose concentration by analyzing and computing an absorbance spectrum obtained on the basis of detection signals resulting from photoelectric conversion by the photoelectric conversion means.
(2) A non-invasive spectrophotometric blood glucose determination apparatus comprising:
   1) near-infrared light dividing means for dividing near-infrared light of 0.8 to 2.5 µm in wavelength, the means consisting of a high frequency generating unit, a piezo element to which a high frequency generated by the high frequency generating unit is applied, an acousto-optic variable oscillation filter to which acoustic vibration is applied from the piezo element, and a light source for supplying light to be brought to incidence on the acousto-optic variable oscillation filter;
   2) near-infrared irradiating means for irradiating a human body with the divided near-infrared lights;
   3) photoelectric conversion means for reflecting with a reflector plate, installed on the back side of the human body, the light transmitted by the human body, to cause the human body to transmit the light again, receiving the light with a light receiving element and photoelectrically converting it; and
   4) glucose concentration computing means for assessing a glucose concentration by analyzing and computing an absorbance spectrum obtained on the basis of detection signals resulting from photoelectric conversion by the photoelectric conversion means.
(3) A spectrophotometric blood glucose determination apparatus comprising:
   1) near-infrared light dividing means for dividing near-infrared light of 0.8 to 2.5 µm in wavelength, the means consisting of a high frequency generating unit, a piezo element to which a high frequency generated by the high frequency generating unit is applied, an acousto-optic variable oscillation filter to which acoustic vibration is applied from the piezo element, and a light source for supplying light to be brought to incidence on the acousto-optic variable oscillation filter;
   2) near-infrared irradiating means for irradiating a blood sample taken from a human body with the divided near-infrared light;
   3) photoelectric conversion means for reflecting with a reflector plate, installed on the back side of the blood sample, the light transmitted by the blood sample, to cause the blood sample to transmit the light again, receiving the light with a light receiving element and photoelectrically converting it; and
   4) glucose concentration computing means for assessing a glucose concentration by analyzing and computing an absorbance spectrum obtained on the basis of detection signals resulting from photoelectric conversion by the photoelectric conversion means.
(4) A non-invasive spectrophotometric blood glucose determination method, comprising:
   1) a near-infrared light dividing step at which a high frequency generated by the high frequency generating unit is applied to a piezo element, acoustic vibration is applied from the piezo element to an acousto-optic variable oscillation filter, light is brought to incidence on the acousto-optic variable oscillation filter, and near-infrared light of 0.8 to 2.5 µm in wavelength is divided;
   2) a near-infrared irradiating step of irradiating a human body with the near-infrared light;
   3) a photoelectric conversion step at which irradiation with the near-infrared light is accomplished, and the light transmitted by the human body is received with a light receiving element and photoelectrically converted; and
   4) a glucose concentration computing step of assessing a glucose concentration by analyzing and computing an absorbance spectrum obtained on the basis of detection signals resulting from photoelectric conversion at the photoelectric conversion step.
(5) A non-invasive spectrophotometric blood glucose determination method, comprising:
   1) a near-infrared light dividing step at which a high frequency generated by the high frequency generating unit is applied to a piezo element, acoustic vibration is applied from the piezo element to an acousto-optic variable oscillation filter, light is brought to incidence on the acousto-optic variable oscillation filter, and near-infrared light of 0.8 to 2.5 µm in wavelength is divided;
   2) a near-infrared irradiating step of irradiating a human body with the near-infrared light;
   3) a photoelectric conversion step of reflecting with a reflector plate, installed on the back side of the human body, the light irradiating and transmitted by the human body, to cause the human body to transmit the light again, receiving the light with a light receiving element and photoelectrically converting it; and
   4) a glucose concentration computing step of assessing a glucose concentration by analyzing and computing an absorbance spectrum obtained on the basis of detection signals resulting from photoelectric conversion at the photoelectric conversion step.
(6) A spectrophotometric blood glucose determination method, comprising:
   1) a near-infrared light dividing step at which a high frequency generated by the high frequency generating unit is applied to a piezo element, acoustic vibration is applied from the piezo element to an acousto-optic variable oscillation filter, light is brought to incidence on the acousto-optic variable oscillation filter, and near-infrared light of 0.8 to 2.5 µm in wavelength is divided;
   2) a near-infrared irradiating step of irradiating a blood sample taken from a human body with the divided near-infrared light;
   3) a photoelectric conversion step of reflecting with a reflector plate, installed on the back side of the blood sample, the light transmitted by the blood sample, to cause the blood sample to transmit the light again, receiving the light with a light receiving element and photoelectrically converting it; and
   4) a glucose concentration computing step of assessing a glucose concentration by analyzing and computing an absorbance spectrum obtained on the basis of detection signals resulting from photoelectric conversion at the photoelectric conversion step.

### Embodiments

The present invention will be described in more specific terms below with reference to preferred embodiments thereof, though the invention is not limited to these embodiments.

### First Embodiment

A blood glucose determination apparatus [a modified version of a Pluscan SH™ (a portable near-infrared spectrophotometer) manufactured by Opto Giken Ltd.] fabricated by using a high frequency electric power source (supplying a high frequency of 50 MHz), a piezo element (PbS) and an acousto-optic variable oscillation filter (AOTF) (a product of IFS (Maryland, U.S.A.)), a light receiving element (PbS), an absorbance spectrum waveform analyzing circuit and an arithmetic circuit in accordance with the hardware configuration of FIG. 1, was connected to a pair of clippers with an optical fiber (see FIG. 2).

Both blocks B and C of the clippers were opened in their respective finger clamping sections; an index finger was inserted into the finger clamp from the under side (the block C side); its second and third joints were fixed in the positions of blocks B and C, respectively; block B was closed; part of the epidermis and the flesh of the finger was clamped in the gap (4 to 6 mm) between blocks A and B; and near-infrared light of 0.7 to 2.5 µm in wavelength was emitted from an irradiating spot to irradiate the clamped portion thereby to carry out the first stage of measurement. Then, block C was closed to choke the blood and, after waiting a few seconds till the measured portion was congested with blood, the second stage of measurement was carried out. FIG. 9 illustrates a near-infrared absorbance spectrum a in a normal state of blood circulation, a near-infrared absorbance spectrum b in a hemostatic state, and a blood spectrum of (b - a = c), i. e. the balance of subtracting a from b, obtained by the first and second stages of measurement. The measured data of this "blood spectrum" c were expanded (digital signals were amplified), resulting in a clearer "blood spectrum" permitting distinction of variations in more detail with more conspicuous relative heights as shown in FIG. 10.

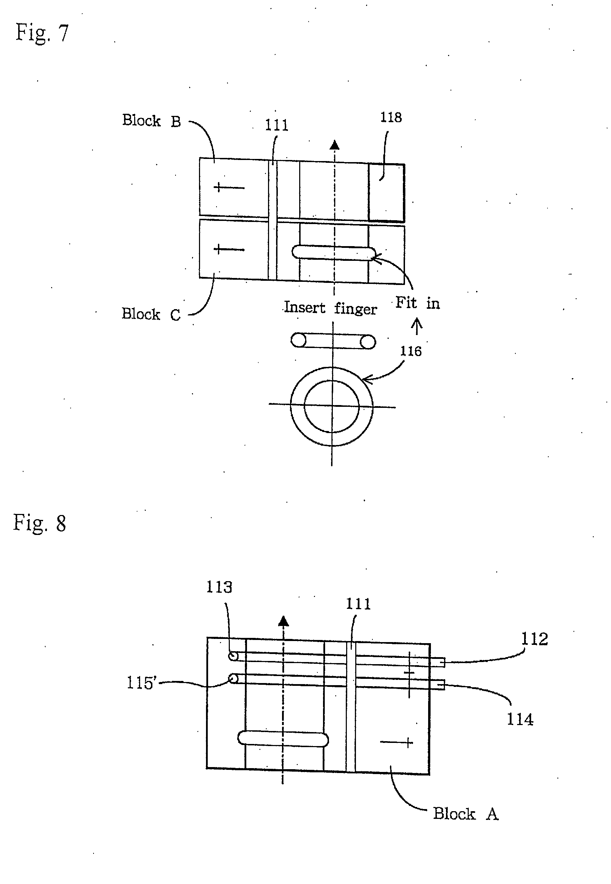

From this "blood spectrum", absorbances at 1,452 µm, 1,948 µm and five other wavelengths (1,614 µm, 1,686 µm, 1,737 µm, 2,067 µm and 2,193 µm) were selected, and those absorbances were converted into glucose concentrations. Measurement was done three times and, separately from that, an absorbance spectrum was determined for each of A (98.8±0.2 mg/dl) and E (181.0±0.2 mg/dl) as shown in FIG. 8. An average of 110.3±0.5 mg/dl was obtained with reference to the difference between A and E at a wavelength of 1,432 µm.

The glucose concentration of another blood sample taken from the same person was measured by the enzymatic method with "Antosense II" (small electrode type blood glucose meter) manufactured by Daikin Industries, Ltd., and determined to be 110 mg/dl. The glucose concentration determined by absorbance measurement with an acousto-optic variable oscillation filter-based spectrophotometric measurement apparatus according to the invention gave a result close to the glucose concentration determined by the enzymatic method.

### Second Embodiment

The glucose concentrations (1) of five persons A through E, including diabetic patients were measured five times each using the blood glucose determination apparatus, which is the first embodiment of the invention in the same operational procedure and under the same measuring conditions as for the first embodiment, and the former of the following results were obtained. The glucose concentrations (2) of the blood samples taken from the same five persons were also measured five times for each by the enzymatic method with "Antosense II" manufactured by Daikin Industries, Ltd., and the latter of the following results were obtained. The glucose concentrations determined by absorbance measurement with the measurement apparatus according to the invention gave results close to the glucose concentrations determined by the enzymatic method. The results obtained with the measurement apparatus according to the invention were found excellent in reproducibility, too.

| | Glucose concentration (1) |
|---|---|
| A | 98.8±0.2 mg/dl |
| B | 102.3±0.2 mg/dl |
| C | 130.2±0.4 mg/dl |
| D | 142.7±0.1 mg/dl |
| E | 181.0±0.2 mg/dl |

| | Glucose concentration (2) |
|---|---|
| A | 99 mg/dl |
| B | 105 mg/dl |
| C | 130 mg/dl |
| D | 143 mg/dl |
| E | 183 mg/dl |

### Third Embodiment

Using an acousto-optic variable oscillation filter (AOTF) (a product of IFS (Maryland, U.S.A.)) as in the first embodiment and the same hardware configuration as what is illustrated in FIG. 1, blood was drawn from a finger tip of the same person as for the first embodiment, and placed directly on a reflector plate (made of silica) to be measured. A blood glucose count of 110.3 mg/dl was obtained. This result demonstrates that the spectrophotometric measurement apparatus according to the invention can be used not only for non-invasive measurement of blood glucose in a portion of a human body but also for quantitative determination of the glucose concentration in blood directly extracted from a body.

When the same blood sample was put into a quartz glass cuvette for absorbance measurement and measurement was carried out by using only transmitted light, a blood glucose count of 110.3 mg/dl was obtained.

As hitherto described, since the present invention uses an acousto-optic variable oscillation filter as one of its constituent elements, it is possible to divide near-infrared light into fine wavelength portions for use in irradiation. Therefore, multiple point measurement is made possible to enable absorbance by intact versions of glucose as well, resulting in a high level of accuracy and reproducibility. Moreover, as it uses no enzymatic reaction, there is no need to use any equipment requiring the setting of complex reaction conditions, and accordingly it is made possible to provide a portable measuring instrument capable of performing measurement by only a simple operation. In addition, since it allows non-invasive measurement, there is no risk of infection including any medical disposables, and the glucose concentration can be measured in a clean state.

Furthermore, since a measuring speed of a few thousand points per second can be achieved, measurement takes only two to three minutes per sample, resulting in a level of efficiency more than five times that of the enzymatic method.

## Claims

1. A spectrophotometric blood glucose determination apparatus capable of measuring the concentration of glucose in blood, comprising:
(1) near-infrared irradiating means for continuously dividing the wavelengths of near-infrared light in a wavelength range of 0.8 to 2.5 µm into fine portions and irradiating a subject of measurement therewith;
(2) photoelectric conversion means for receiving and photoelectrically converting the lights transmitted or reflected by said subject of measurement irradiated therewith by said near-infrared irradiating means; and
(3) glucose concentration computing means for determining the glucose concentration in the blood within said subject of measurement by analyzing the absorbance spectrum obtained on the basis of detection signals resulting from photoelectric conversion by said photoelectric conversion means.

2. The spectrophotometric blood glucose determination apparatus as set forth in claim 1, in which said near-infrared irradiating means is a near-infrared spectrophotometric unit, comprising:
(i) a light source;
(ii) an acousto-optic variable oscillation tunable filter, on which light comes incident from said light source, for emitting near-infrared light in the wavelength range of 0.8 to 2.5 µm;
(iii) a high frequency vibrator for applying acoustic vibration to said acousto-optic tunable filter; and
(iv) a high frequency generating unit for applying a high frequency to said high frequency vibrator.

3. The spectrophotometric blood glucose determination apparatus as set forth in claim 2, wherein the medium of said acousto-optic tunable filter is a birefringent crystalline material.

4. The spectrophotometric blood glucose determination apparatus, as set forth in one of the preceding claims, wherein said photoelectric conversion means is a photoelectric conversion unit consisting of a light receiving element for receiving the light transmitted by the subject of measurement and supplying detection signals giving an absorbance spectrum.

5. The spectrophotometric blood glucose determination apparatus as set forth in one of the preceding claims, wherein said glucose concentration computing means comprises an absorbance spectrum waveform analyzing unit to which detection signals from said light receiving element are supplied and a glucose concentration computing unit for converting the absorbance spectrum into glucose concentrations.

6. The spectrophotometric blood glucose determination apparatus as set forth in claim 5, wherein said absorbance spectrum is a blood spectrum resulting from the subtraction of a normal state spectrum from a hemostatic state spectrum figured out by an arithmetic circuit in an image processing manner.

7. The spectrophotometric blood glucose determination apparatus as set forth in claim 5 or 6, wherein wavelengths selected in said absorbance spectrum are converted values based on five or more wavelengths at 1.44 µm, 1.94 µm and in a 0.8 to 2.5 µm band.

8. The spectrophoto metric blood glucose determination apparatus as set forth in one of the preceding claims, wherein said subject of measurement is part of a human body whose glucose concentration in blood is measurable.

9. The spectrophotometric blood glucose determination apparatus as set forth in one of the preceding claims, wherein fixing means for said subject of measurement is capable of giving rise to blood congestion by holding with pressure the position to be measured of a human body.

10. A spectrophotometric blood glucose determination method permitting determination of a concentration of glucose in blood, comprising:
(1) a near-infrared irradiating step of continuously dividing wavelengths of near-infrared light in a wavelength range of 0.8 to 2.5 µm into fine portions and irradiating a subject of measurement therewith;
(2) a photoelectric conversion step of receiving and photoelectrically converting the lights transmitted or reflected by said subject of measurement irradiated therewith at said near-infrared irradiating step; and
(3) a glucose concentration computing step of assessing the glucose concentration in the blood within said subject of measurement by analyzing and computing an absorbance spectrum obtained on the basis of detection signals resulting from photoelectric conversion at said photoelectric conversion step.

11. The spectrophotometric blood glucose determination method as set forth in Claim 10, wherein said near-infrared irradiating step is a combination of:
(i) a sub-step of applying a high frequency onto a high frequency vibrator;
(ii) a sub-step at which the high frequency vibrator to which the high frequency was applied at said sub-step (i) applies acoustic vibration to an acousto-optic tunalbe filter; and
(iii) a sub-step of bringing light incident on an acousto-optic tunable filter, to which acoustic vibration is applied at said sub-step (ii), from a light source, and causing near-infrared light in the wavelength range of 0.8 to 2.5 µm to be emitted.

12. The spectrophotometric blood glucose determination method as set forth in Claim 10 or 11, wherein the medium of said acousto-optic tunable filter is a birefringent crystalline material.

13. The spectrophotometric blood glucose determination method as set forth in one of the preceding claims 10 to 12, wherein said photoelectric conversion step is a combination of a sub-step of supplying light transmitted or reflected by the subject of measurement to a light receiving element and a sub-step at which the light receiving element supplies detection signals giving an absorbance spectrum.

14. The spectrophotometric blood glucose determination method as set forth in one of the preceding claims 10 to 13, wherein said glucose concentration computing step is a combination of an absorbance spectrum waveform analyzing step to which detection signals from said light receiving element are supplied and a glucose concentration computing step of converting the absorbance spectrum into glucose concentrations.

15. The spectrophotometric blood glucose determination method as set forth in one of the preceding claims 10 to 14, wherein said absorbance spectrum is a blood spectrum resulting from the subtraction of a normal state spectrum from a hemostatic state spectrum figured out by an arithmetic circuit in an image processing manner.

16. The spectrophotometric blood glucose determination method as set forth in one of the preceding claims 10 to 15, wherein wavelengths selected in said absorbance spectrum are converted values based on five or more wavelengths at 1.44 µm, 1.94 µm and in a 0.8 to 2.5 µm band.

17. The spectrophotometric blood glucose determination method as set forth in one of the preceding claims 10 to 16, wherein said subject of measurement is part of a human body whose glucose concentration in blood is measurable.

18. The spectrophotometric blood glucose determination method as set forth in one of the preceding claims 10 to 16, wherein said subject of measurement is a blood-congested part resulting from the holding of the measured region of a human body with pressure.
